# EUROPEAN PATENT APPLICATION

(11) **EP 4 137 515 A1**
(43) Date of publication of application: **22.02.2023**
(21) Application number: 21192286.9
(22) Date of filing: 20.08.2021
(51) Int. Cl.: C07K 16/28, C07K 16/40, A61P 35/00, G01N 33/574

(54) **ANTIGEN-BINDING POLYPEPTIDES AGAINST CD73**

(71) Applicant: Universitätsklinikum Hamburg-Eppendorf, 20246 Hamburg (DE)
(72) Inventor: NOLTE, Friedrich, 20457 Hamburg (DE); MENZEL, Stephan, 20253 Hamburg (DE); MAGNUS, Tim, 22455 Hamburg (DE); HAAG, Friedrich, 20251 Hamburg (DE); DUAN, Yinghui, 20246 Hamburg (DE); RISSIEK, Björn, 22523 Hamburg (DE)
(74) Representative: Uexküll & Stolberg

(57) **Abstract**

The invention relates to antigen-binding polypeptides comprising the CDR1, CDR2 and CDR3 region of a VHH domain of a camelid heavy chain antibody. The polypeptides bind specifically to CD73 and are therefore suitable for the diagnosis as well as for the therapeutic and prophylactic treatment of diseases characterised by increased CD73 expression. Conjugates and pharmaceutical compositions comprising the antigen-binding polypeptides are also disclosed. In addition, the invention relates to the use of such antigen-binding polypeptides in methods for detecting CD73 or CD73-expressing cells in a biological sample. Methods for purifying and concentrating CD73 or CD73-expressing cells using the antigen-binding polypeptides are also described.

## Description

The invention relates to antigen-binding polypeptides comprising the CDR1, CDR2 and CDR3 region of a VHH domain of a camelid heavy chain antibody. The polypeptides bind specifically to CD73 and are therefore suitable for immunotherapy that includes the blocking of CD73 signaling. In particular, the polypeptides are suitable for the therapeutic and prophylactic treatment of certain diseases that benefit for interfering with the enzymatic activity of CD73. In addition, the polypeptides are suitable for the diagnosis as well as for the therapeutic and prophylactic treatment of diseases characterised by increased CD73 expression. Conjugates and pharmaceutical compositions comprising the antigen-binding polypeptides are also disclosed. In addition, the invention relates to the use of such antigen-binding polypeptides in methods for detecting CD73 or CD73-expressing cells in a biological sample. Methods for purifying and concentrating CD73 or CD73-expressing cells using the antigen-binding polypeptides are also described.

### BACKGROUND

The search for new therapeutic targets has led to the identification of immunosuppressive adenosine as an important regulator of antitumor immunity. Selective inhibitors targeting various components of the adenosinergic pathway have been contemplated for use in cancer therapy. Components of the adenosinergic pathway which are of particular interest in this respect include CD39 and CD73.

CD39 is an integral membrane protein that catalyzes the phosphohydrolysation of adenosine triphosphate (ATP). The human protein is predicted to include 510 amino acids, seven potential N-linked glycosylation sites and two transmembrane regions. It encompasses a large extracellular domain that consists of five conserved domains which is referred to as apyrase conserved regions (ACR) 1-5 in the literature and which are important for the catabolic activity of the enzyme. It has been reported that CD39 is constitutively expressed in spleen, thymus, lung, and placenta. Its expression is regulated by several pro-inflammatory cytokines, oxidative stress and hypoxia. In addition, a significantly increase in the expression of CD39 has been found in chronic lymphocytic leukemia and in a number of solid tumors, including colorectal cancer and pancreatic cancer which indicates that this enzyme is involved in the development and progression of these diseases.

CD73 is an ecto-5'-nucleotidase (NT5E) which catalyzes the dephosphorylation of adenosine monophosphate (AMP) to adenosine, the latter of which suppresses immune activation through the A2A receptor. The mature form of CD73 consists of 548 amino acids and has an estimated molecular mass of 61 kDa. CD73 is expressed in a variety of tissues, including colon, brain, kidney, liver, lung, and heart. It has also been reported that the enzyme can be found on endothelial cells and on leukocytes derived from peripheral blood, spleen, lymph nodes, thymus and bone marrow. It appears that CD73 expression is upregulated under hypoxic conditions and in the presence of pro-inflammatory mediators, such as transforming growth factor (TGF)-β, interferons (IFNs), tumor necrosis factor (TNF)-α, and interleukin (IL)-1β. Increased levels of CD73 expression has also been reported in several types of cancer, where elevated CD73 levels correlate with poor clinical outcomes. This suggests that CD73 is involved in the onset and progression of neoplasia.

Accordingly, CD39 and CD73 represent interesting targets for the diagnosis and therapy of various cancer diseases. Monoclonal antibodies against CD73, which can be used in the context of diagnostics and therapy, are known in the state of the art. Human anti-CD73 antibodies are currently being tested in phase I clinical trials for treating advanced solid tumors. However, the treatment of tumors with monoclonal antibodies is regularly associated with considerable disadvantages. For example, monoclonal antibodies often show an insufficient stability after administration into a patient. In addition, the costs involved in the production of monoclonal antibodies are very high. Specifically, the production of humanised antibodies with decreased immunogenicity is laborious and cost-intensive. In addition, owing to their size, complete antibodies show limited tissue permeability.

In the state of the art, various strategies have been developed to solve the problems associated with the administration of antibodies. For example, fragments of full IgG antibodies are discussed for therapeutic purposes. However, these fragments often show reduced specificity and affinity with respect to the antigen compared to the complete antibody. Furthermore, these fragments often show only low solubility in aqueous solutions.

It has been described in the art that camelid antibodies have properties that render them particularly suitable for therapeutic use in humans. In addition to conventional antibodies, which each have two heavy and two light chains, camelids also produce antibodies that exclusively consist of heavy chains (14-15). These so-called heavy chain antibodies are homodimers of two identical heavy chains that interact with the antigen through a single variable domain called VHH (variable domain of the heavy chain of heavy chain antibodies).

The VHH domain of a camelid heavy chain antibody has three complementary determining regions (CDRs) and four framework regions (FRs) that alternate with the CDRs in the primary sequence of the VHH domain. The VHH domain of a heavy chain antibody hence forms a small polypeptide unit with high antigen-binding capacity. Due to an exceptionally long CDR3 region with a length of 7-25 amino acids, the VHH domain can bind in cavities of protein antigens, unlike conventional antibodies, and thus block the active site of an enzyme. VHH domains can be produced recombinantly as soluble proteins in bacteria or mammalian cells. Such recombinantly produced VHH domains are also called single-domain antibodies (sdABs) or nanobodies. Currently, several nanobodies are being tested in phase II clinical trials.

VHH single domain antibodies are more stable and about ten times smaller than conventional antibodies and therefore exert a high solubility and significantly improved tissue penetration properties. For this reason, VHH single domain antibodies have been proposed for the treatment of various diseases.

The present invention provides for the first time VHH single domain antibodies capable of specifically binding to CD73. Such VHH single domain antibodies directed against CD73 have not been known in the prior art. The single domain antibodies of the present invention are particularly suitable for immunotherapy that includes the blocking of CD73 signaling. In particular, the polypeptides are suitable for the therapeutic and prophylactic treatment of certain diseases that benefit for interfering with the enzymatic activity of CD73 that occurs on cells, on microvesicles or in soluble form. In addition, the polypeptides are suitable for use in diagnosis and the therapeutic treatment of diseases characterised by increased expression of CD73.

### DESCRIPTION OF THE INVENTION

In the present invention, heavy chain antibodies were first generated by immunising alpacas with human or murine CD73. Subsequently, RNA was isolated from peripheral blood lymphocytes of the alpacas and converted into cDNA by reverse transcription. Based on the isolated RNA, the variable domains of the heavy chain antibodies were amplified by PCR. Phage display libraries were prepared by cloning the VHH fragments into a phagemid vector. These libraries were selected using CD73-transfected HEK cells in a panning procedure. The sequences of the selected single domain antibodies were then determined by sequencing and recombinantly expressed in HEK-6E cells. In this way, the present invention provides VHH single domain antibodies suitable for use in therapeutic and diagnostic methods. In addition, the present invention provides CDR regions from VHH single domain antibodies that can be used in the construction of recombinant antigen-binding polypeptides.

Accordingly, in a first aspect, the invention provides an antigen-binding polypeptide comprising the complementarity-determining regions CDR1, CDR2 and CDR3 of a VHH domain of a camelid heavy chain antibody necessary for binding of a VHH single domain antibody, wherein the polypeptide specifically binds to CD73.

The term "CD73" as used herein refers to the "cluster of differentiation protein 73". In a preferred embodiment, the protein to which the polypeptide of the invention binds is human CD73 (gene ID 4907) which is referred to herein as SEQ ID NO:84. In a particularly preferred embodiment, the polypeptide of the invention specifically binds to an epitope that is located in the C-terminal domain of CD73 that contains the substrate binding site and dimerization interface of the nucleotidase molecule. The C-terminal domain of CD73 extends from amino acid 337 to amino acid 549.

The polypeptides according to the invention comprise CDR regions derived from heavy chain antibodies of camelids. Heavy chain antibodies are a particular group of immunoglobulins consisting exclusively of heavy chains. In addition to two constant domains, a heavy chain of such an antibody contains a single variable domain, which in turn consists of three CDR regions and four FR regions. Interaction with the antigen takes place exclusively via this variable domain, which is also referred to as the VHH domain. Due to distinctive loop structures in the CDR3, a VHH domain is also able to recognise cryptic epitopes. Heavy chain antibodies are produced in only a few species, e.g. in llamas and alpacas. The camelid group is a family of mammals from the order Artiodactyla (even-toed ungulates). The camelid family includes camels, dromedaries, llamas, alpacas, guanacos and vicuñas. In a particularly preferred embodiment of the invention, the antigen-binding polypeptide comprises CDR regions derived from a VHH domain of a llama or alpaca, and more preferably an alpaca.

The polypeptide according to the invention has a specific affinity for CD73, and more preferably human CD73. This means that a polypeptide according to the invention binds to CD73 with a binding affinity that is significantly higher than the binding affinity for binding to another protein that is not homologous to the sequence of CD73.

Preferably, the binding affinity of the polypeptide according to the invention is at least 2-fold, at least 5-fold, at least 10-fold, at least 20-fold, at least 50-fold, at least 75-fold, at least 100-fold, at least 150-fold, at least 200-fold, at least 250-fold, at least 500-fold, at least 600-fold, at least 700-fold, at least 800-fold, at least 900-fold, at least 1000-fold, at least 2000-fold, at least 5000-fold, or at least 10000-fold higher than the binding affinity for a protein not homologous to CD73, such as human albumin.

The binding affinity of the polypeptide according to the invention will preferably bind to its antigen with a dissociation constant (Kd) of 10⁻⁵ M or less, and more preferably in the range of 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. Accordingly, the polypeptide according to the invention will preferably bind to its antigen with a dissociation constant (Kd) of 10⁻⁵ M to 10⁻¹² M, and more preferably 10⁻⁶ M to 10⁻¹² M, 10⁻⁷ M to 10⁻¹² M, 10⁻⁸ M to 10⁻¹² M, or 10⁻⁹ M to 10⁻¹² M. A Kd value of greater than 10⁻⁴ M is generally considered to indicate non-specific binding. Methods for measuring the binding affinities of antibodies are well known in the prior art and include, for example, the determination of Kd values by flow cytometry, surface plasmon resonance spectroscopy or microscale thermophoresis.

In a particularly preferred embodiment of the invention, the CD73-specific antigen-binding polypeptide comprises the CDR regions CDR1, CDR2 and CDR3 shown in SEQ ID NO:1-3. Even more preferably, the antigen-binding polypeptide comprises
(a) the CDR sequences shown in SEQ ID NO:4-6;
(b) the CDR sequences shown in SEQ ID NO:7-9;
(c) the CDR sequences shown in SEQ ID NO: 10-12;
(d) the CDR sequences shown in SEQ ID NO: 13-15;
(e) the CDR sequences shown in SEQ ID NO:16-18;
(f) the CDR sequences shown in SEQ ID NO: 19-21,
(g) the CDR sequences shown in SEQ ID NO:22-24,
(h) the CDR sequences shown in SEQ ID NO:25-27,
or CDR sequences which differ from the CDR sequences shown in (a)-(h) in not more than one amino acid per CDR region.

In another particularly preferred embodiment of the invention, the CD73-specific antigen-binding polypeptide comprises the CDR regions CDR1, CDR2 and CDR3 shown in SEQ ID NO:28-30. Even more preferably, the antigen-binding polypeptide comprises
(a) the CDR sequences shown in SEQ ID NO:31-33;
(b) the CDR sequences shown in SEQ ID NO:34-36;
(c) the CDR sequences shown in SEQ ID NO:37-39;
or CDR sequences which differ from the CDR sequences shown in (a)-(c) in not more than one amino acid per CDR region.

In another particularly preferred embodiment of the invention, the CD73-specific antigen-binding polypeptide comprises the CDR regions CDR1, CDR2 and CDR3 shown in SEQ ID NO:40-42. Even more preferably, the antigen-binding polypeptide comprises
(a) the CDR sequences shown in SEQ ID NO:43-45;
(b) the CDR sequences shown in SEQ ID NO:46-48;
(c) the CDR sequences shown in SEQ ID NO:49-51;
(d) the CDR sequences shown in SEQ ID NO:52-54,
(e) the CDR sequences shown in SEQ ID NO:55-57,
(f) the CDR sequences shown in SEQ ID NO:58-60;
or CDR sequences which differ from the CDR sequences shown in (a)-(f) in not more than one amino acid per CDR region.

It is apparent to the skilled person that within the presently disclosed CDRs of SEQ ID NO:4-27, SEQ ID NO:31-39, SEQ ID NO:43-60 and SEQ ID NO:78-80, some modification of the amino acid sequence is possible without affecting the binding ability of the VHH single domain antibody. For example, it may be possible to modify CDR1 of the single domain antibody by substituting 1, 2, or 3 amino acids. Similarly, it may be possible to modify CDR2 of the single domain antibody by substituting 1 or 2 amino acids. Due to the length of CDR3, it may be possible to modify this CDR by substituting 1, 2, 3, 4, 5, or 6 amino acids.

In general, any of the amino acid residues within the CDRs shown in SEQ ID NO:4-27, SEQ ID NO:31-39, SEQ ID NO:43-60 and SEQ ID NO:78-80 may be replaced with another residue as long as the resulting CDR is still able (along with the other two CDRs) to specifically bind to CD73. Furthermore, 1, 2 or 3 amino acids within one of the sequences shown in SEQ ID NO:4-27, SEQ ID NO:31-39, SEQ ID NO:43-60 and SEQ ID NO:78-80 may also be deleted or added by addition as long as the ability to bind to CD73 is not completely eliminated.

Thus, also encompassed by the invention are antigen-binding polypeptides having CDRs that differ from the sequences shown in SEQ ID NO:4-27, SEQ ID NO:31-39, SEQ ID NO:43-60 and SEQ ID NO:78-80 in individual amino acid positions. The invention therefore specifically relates to antigen-binding polypeptides having CDR sequences that differ from any of the CDR sequences shown in SEQ ID NO:4-27, SEQ ID NO:31-39, SEQ ID NO:43-60 and SEQ ID NO:78-80 in not more than a single amino acid per CDR.

In a further preferred embodiment, the antigen-binding polypeptide of the invention comprises a complete VHH domain of a camelid heavy chain antibody. Such a VHH domain, when used alone, is capable of binding to the corresponding antigen and is therefore also referred to herein as a "VHH single domain antibody". In addition to the three CDR regions responsible for antigen binding, the VHH domain also comprises the four framework regions FR1-FR4, which are located between the CDR regions. The scaffold regions may be fully conserved in the VHH domain of the invention, or they may be in the form of fragments of the original scaffold sequences. For example, the scaffold sequences delimiting the VHH domain C-terminally and N-terminally may be truncated by one or more amino acids. Such modified VHH domains are expressly encompassed by the invention. As used herein, "VHH single domain antibody" means an antigen-binding polypeptide that lacks the constant domains CH2 or CH3 compared to a full heavy chain antibody. In a preferred embodiment, the antigen-binding polypeptide of the invention is a VHH single domain antibody.

Preferred antigen-binding polypeptides comprising a VHH domain of a camelid heavy chain antibody and specifically binding to CD73 comprise or consist of the sequences depicted in SEQ ID NO:61-77 and SEQ ID NO:81-83. The invention further extends to variants of the polypeptides shown in SEQ ID NO:61-77 and SEQ ID NO:81-83 that are homologous to the polypeptides shown in SEQ ID SEQ ID NO:61-77 and SEQ ID NO:81-83 and also have the ability to specifically bind CD73. As used herein, the term "variant" means an amino acid sequence that has been modified by deletion, substitution or addition of amino acids such that it has at least 80%, 85%, 90%, 91%, 92%, 93%, 94%, 95%, 96%, 97%, 98%, 99% sequence identity to any of the sequences shown in SEQ ID NO:61-77 and SEQ ID NO:81-83 when those sequences are optimally aligned, e.g., with the GAP or BESTFIT programs using the default gap procedure. Computer programs for determining amino acid sequence identity are sufficiently known in the prior art.

VHH single domain antibodies are distinguished from conventional mammalian antibodies in particular by the fact that only three CDRs are required for binding the antigen. The invention therefore also relates to those antigen-binding polypeptides which comprise only the CDR1, CDR2 and CDR3 regions required for binding CD73 of one of the polypeptides exemplified in SEQ ID NO:61-77 and SEQ ID NO:81-83, wherein the regions located between the CDR regions show only minor or essentially no similarity to the corresponding regions of one of the polypeptides shown in SEQ ID NO:61-77 and SEQ ID NO:81-83.

Thus, the framework regions (FR1-FR4) of the polypeptides provided herein can differ from that of the VHH single domain antibodies shown in SEQ ID NO:61-77 and SEQ ID NO:81-83 without adversely affecting the binding of the polypeptides. Preferably, the variants differ from the polypeptides shown in SEQ ID NO:61-77 and SEQ ID NO:81-83 only by the exchange of a few amino acids. Particularly preferably, the variants of the polypeptides shown in SEQ ID NO:61-77 and SEQ ID NO:81-83 differ from the polypeptides shown in SEQ ID NO:61-77 and SEQ ID NO:81-83 in less than 25, in less than 20, in less than 15, in less than 10, or in less than 5 amino acids. Preferably, variants of the polypeptides shown in SEQ ID NO:61-77 and SEQ ID NO:81-83 differ from the polypeptides shown in SEQ ID NO:61-77 and SEQ ID NO:81-83 in 5, 4, 3, 2 or only 1 amino acid position.

In one aspect, the invention thus relates to a CD73-specific antigen-binding polypeptide comprising an amino acid sequence selected from the group consisting of:
(a) the amino acid sequences of SEQ ID NO:61-77 and SEQ ID NO:81-83; and
(b) an amino acid sequence having at least 80% or more sequence identity to one of the amino acid sequences of SEQ ID NO:61-77 and SEQ ID NO:81-83

Preferably, the substitutions that distinguish the variants from the VHH single domain antibodies shown in SEQ ID NO:61-77 and SEQ ID NO:81-83 are conservative substitutions, i.e. substitutions of one or more amino acid residues by an amino acid of similar polarity acting as a functional equivalent. Preferably, the amino acid residue serving as a substitution is selected from the same group of amino acids as the amino acid residue to be substituted. For example, a hydrophobic residue may be replaced by another hydrophobic residue, or a polar residue may be replaced with another polar residue having the same charge. Functionally homologous amino acids which may be used for conservative substitution include, for example, non-polar amino acids such as glycine, valine, alanine, isoleucine, leucine, methionine, proline, phenylalanine, and tryptophan. Examples of uncharged polar amino acids include serine, threonine, glutamine, asparagine, tyrosine, and cysteine. Examples of charged polar (acidic) amino acids include histidine, arginine, and lysine. Examples of charged polar (basic) amino acids include aspartic acid and glutamic acid.

Furthermore, variants of the sequences shown in SEQ ID NO:61-77 and SEQ ID NO:81-83also include polypeptides in which one or more amino acids have been inserted into the amino acid sequence of one of the polypeptides shown in SEQ ID NO:61-77 and SEQ ID NO:81-83. Such insertions can occur at any position of the polypeptides shown in SEQ ID NO:61-77 and SEQ ID NO:81-83, as long as the resulting variant of the polypeptide retains its immunological activity (i.e. the ability to specifically bind CD73). Furthermore, proteins shown in SEQ ID NO:61-77 and SEQ ID NO:81-83 in which one or more amino acids within the sequence are missing compared to the reference polypeptide are also considered variants of the polypeptide shown herein. Such deletions may affect any amino acid positions within the sequences shown in SEQ ID NO:61-77 and SEQ ID NO:81-83. In particular, the deletions may be those involving two or more (e.g. 3, 4 or 5) consecutive amino acids from any of the sequences of SEQ ID NO:61-77 and SEQ ID NO:81-83. Preferably, fewer than 25, fewer than 20, fewer than 15, fewer than 10, fewer than 5 amino acid positions in total have been inserted or deleted in the variant compared to the respective reference polypeptide having one of the sequences shown in SEQ ID NO:61-77 and SEQ ID NO:81-83.

Also included in the invention are immunologically active fragments of the polypeptides (or variants thereof) shown in SEQ ID NO:61-77 and SEQ ID NO:81-83. Immunologically active fragments are understood herein to be sequences which differ from the amino acid sequences shown in SEQ ID NO:61-77 and SEQ ID NO:81-83or variants thereof by the absence of one or more amino acids at the N-terminus and/or at the C-terminus of the protein and which are still capable of specifically binding to CD73, preferably human CD73. For example, immunologically active fragments of the sequence shown in SEQ ID NO:61 may be fragments lacking the first two N-terminal and/or C-terminal amino acids of the polypeptide shown in SEQ ID NO:61. The skilled person will readily be able to determine the ability of fragments to bind to CD73, e.g. by competitive antibody assays.

The polypeptides shown in SEQ ID NO:61-77 and SEQ ID NO:81-83 and variants or fragments thereof may further be structurally modified at one or more positions in the amino acid sequence, such as by introducing a modified amino acid. According to the invention, these modified amino acids may be amino acids that have been modified by biotinylation, phosphorylation, glycosylation, acetylation, branching and/or cyclisation.

In some embodiments, it is preferred that the variants or fragments of the antigen-binding polypeptides of the invention retain more than 50% of the original binding affinity for CD73. It is even more preferred that the variants or fragments retain more than 60%, 70%, 80%, 90%, 95%, 96%, 97%, 98% or 99% of the original binding affinity of the antigen-binding polypeptides of the invention, e.g. the polypeptides shown in SEQ ID NO:61-77 and SEQ ID NO:81-83. The binding affinity for CD73 can be measured using *in vitro* assays. In other embodiments, it may be preferred that the variants or fragments of the antigen-binding polypeptides of the invention have a lower binding affinity of less than 50% of the original binding affinity for CD73. Such variants and fragments are preferably used in a multivalent targeting approach using e.g. a bispecific or multispecific polypeptide construct.

In another aspect, the invention provides a nucleic acid molecule encoding an antigen-binding polypeptide as defined above. The nucleic acid molecule may be DNA or RNA. Preferably, it is single-stranded or double-stranded DNA, such as genomic DNA or cDNA. Preferably, the polynucleotide encodes a polypeptide comprising one of the amino acid sequences shown in SEQ ID NO:61-77 and SEQ ID NO:81-83 or a variant of any of these.

The invention further comprises an expression vector comprising a nucleic acid molecule encoding an antigen-binding polypeptide of the present invention. Expression vectors are typically DNA constructs that have an origin of replication that allows the vector to replicate independently of the host cell. Expression vectors also include regulatory control elements that are functionally linked to the nucleotide sequences to be expressed and control their transcription and/or translation. Suitable control elements may be prokaryotic promoters (constitutive or regulatable, such as lac, trp, T3, T7 or gpt promoter), eukaryotic promoters (constitutive or regulatable, such as e.g. thymidine kinase promoter, SV40 promoter or CMV promoter), operators, transcription or translation enhancers, transcription or translation terminators, ribosome binding sites, polyadenylation signals, selection markers, silencers and repressor sequences. Numerous prokaryotic and eukaryotic expression vectors suitable for carrying out the present invention are known to those skilled in the art. Examples include the prokaryotic vectors pHEN2, pGEM, pBluescript and pUC, which are regularly used for heterologous expression in *E. coli.* Examples of eukaryotic expression vectors include pcDNA3.1, pOG44, pSV2CAT Rc/CMV, Rc/RSV, GEM1 and the like.

The invention also relates to a host cell comprising a nucleic acid molecule encoding an antigen-binding polypeptide of the present invention or an expression vector comprising such a nucleic acid molecule. In particular, it may be a prokaryotic or a eukaryotic host cell. Examples of host cells that may be used in the context of the invention include prokaryotic host cells, such as bacterial strains of *Escherichia coli*, *Bacillus subtilis* and the like. Suitable eukaryotic cells include yeast cells, insect cells, mammalian cells, plant cells and the like. The antigen-binding polypeptide of the present invention is preferably expressed in mammalian cells, e.g. HEK cells. The method of transforming or transfecting the host cell with an expression vector comprising a nucleic acid molecule encoding the antigen-binding polypeptide will depend on the type of expression vector and the host cell used. The skilled person will have no difficulty in selecting suitable vectors and host cells for recombinant expression.

The invention also provides a method for the recombinant production of an antigen-binding polypeptide of the invention, said method comprising: (a) culturing a host cell as described above under conditions that allow expression of a nucleic acid molecule encoding an antigen-binding polypeptide of the present invention; and (b) isolating the antigen-binding polypeptide from the culture. In the preparation of the antigen-binding polypeptides of the invention, the nucleic acid molecules, expression vectors and host cells described above may be used.

In another aspect, the invention relates to a method for detecting CD73 and/or CD73-expressing cells in a biological sample, comprising the steps of
(a) contacting a CD73-specific antigen-binding polypeptide described above and defined in the claims with the biological sample under conditions that allow the formation of complexes of the antigen-binding polypeptide and CD73; and
(b) detecting the complexes of the polypeptide and CD73/CD73-expressing cells.

The method comprises, as a first step, the contacting of an antigen-binding polypeptide of the invention with the biological sample. This step is carried out under conditions that allow for the formation of binding complexes consisting of the antigen-binding polypeptide and CD73 and/or CD73-expressing cells. Conditions that allow the formation of complexes consisting of the antigen-binding polypeptides of the invention and CD73 are known in the prior art and are sufficiently described in the context of binding of antibodies to protein antigens. Such conditions may include a temperature in the range of 4-37°C, a pH in the range of 6-8 and an incubation time in the range of several minutes to several hours. It will be apparent to one skilled in the art that suitable binding conditions for a given pair of a antigen-binding polypeptide and CD73 will depend on various factors, such as the concentration of the antigen-binding polypeptide, the anticipated concentration of CD73 in the sample, and the like. The skilled person will be able to determine suitable conditions for binding between polypeptide and CD73 based on routine experiments to optimise the formation of the binding complexes.

After the antigen-binding polypeptide has bound to CD73 and/or CD73-expressing cells, the immune complexes consisting of CD73 or CD73-expressing cells and the polypeptide specifically bound to them are detected. For this purpose, well-known methods can be used. For example, the antigen-binding polypeptide according to the invention can be provided with a detectable label prior to contacting the sample suspected of containing the CD73 and/or CD73-expressing cells. The label may be, for example, a fluorescent, chemiluminescent or radioactive label. Labelling with enzymes, such as horseradish peroxidase or alkaline phosphatase, is also possible. Another method that allows detection of the complexes is the recombinant expression of the antigen-binding polypeptides as fusion proteins that have one or more peptide epitopes that can be visualised using labelled antibodies.

In a particularly preferred embodiment of the invention, the method is used for the quantitative detection of CD73 and/or CD73-expressing cells in a biological sample. The quantitative detection can be carried out by methods that have been described in the prior art, for example by quantitative determination of a fluorescent, chemiluminescent or radioactive labelling of polypeptides in complexes. The determination can be carried out by comparison with a reference sample, i.e. by comparison with a sample containing a known amount of CD73 and/or CD73-expressing cells.

In a further aspect, the invention relates to a method for purifying and/or concentrating CD73 and/or CD73-expressing cells, comprising the steps of
(a) contacting a CD73-specific antigen-binding polypeptide as described above and defined in the claims with the biological sample under conditions that allow the formation of complexes of the antigen-binding polypeptide and CD73; and
(b) separating the complexes of the CD73-specific antigen-binding polypeptide and CD73/CD73-expressing cells from the sample.

Preferably, the CD73-specific polypeptide used in the above method is one comprising one or more of the CDR regions listed in SEQ ID NO:1-60 and SEQ ID NO:78-80 or modifications thereof as described above. In a particularly preferred embodiment, the CD73-specific polypeptides comprise the sequences or fragments listed in SEQ ID NO: 1-60 and SEQ ID NO:78-80 or immunologically active fragments or variants thereof as described above.

Purification and/or concentration of CD73 and/or the CD73-expressing cells is preferably performed with antigen-binding polypeptides immobilised on a solid support, such as a material suitable for use in chromatographic separation procedures. Suitable column materials include Sepharose (e.g. Q-Sepharose, DEAE-Sepharose, SP-Sepharose) and the like. The CD73-binding polypeptides may further be immobilised on beads, e.g., agarose or glass beads. Preferably, the beads are magnetisable which facilitates separation of the binding complexes adhering to the beads after incubation in CD73-containing samples. Methods for coupling polypeptides to a solid support are sufficiently known in the prior art. For example, a coupling method may comprise the immobilisation of polypeptides which linked to biotin or derivatives of biotin to a support material that has been previously coated with streptavidin or derivatives thereof. Such compounds are commercially available from various suppliers. Alternatively, the protein may be directly bound to the support material by chemical reaction. The sample containing the CD73 and/or CD73-expressing cells is passed over the support material so that the CD73 and/or CD73-expressing cells are bound by the immobilised antigen-binding polypeptides. By using a suitable wash buffer, all non-specifically bound molecules and cells are washed from the support material while the CD73 and/or CD73-expressing cells are retained by the support material. In a final step, CD73 can be eluted from the material using a suitable elution buffer (e.g. a high salt buffer), thus obtaining the purified and/or concentrated CD73 protein. Alternatively, the CD73 bound to the support material or the CD73-expressing cells can be detected by an immunological detection method.

In some embodiments, the antigen-binding polypeptides are not coupled to a support. In these embodiments, separation from the sample can be achieved, e.g. by designing the CD73-specific antigen-binding polypeptide of the invention as a fusion polypeptide that comprises an affinity tag. Such fusion polypeptides can be efficiently purified using a material that has a particularly high affinity for the affinity tag used. For example, the affinity tag may have 6-12 histidine residues that specifically interact with a chelating nickel ion matrix. Alternatively, the affinity tag may comprise a glutathione S-transferase that can be purified using a glutathione matrix. Numerous other affinity tags are known in the art and can be used in conjunction with the antigen-binding polypeptides of the invention. Examples of affinity tag and affinity ligand pairs include maltose-binding protein (MBP) and maltose; avidin and biotin; streptag and streptavin or neutravidin.

Fusion proteins having an affinity tag may be produced, for example, by ligation of a DNA encoding an antigen-binding polypeptide of the invention with a sequence encoding the affinity tag. In cases where the affinity tag is not a peptide, the affinity tag may be conjugated to the antigen-binding polypeptide by chemical coupling reactions. For example, the antigen-binding polypeptide may be chemically coupled to biotin. Labelling with biotin can also be achieved by attaching to the antigen-binding polypeptide a peptide that is recognised by a biotin-protein ligase (EC 6.3.4.15), such as BirA from *E. coli.* Suitable peptides include the 13 amino acid minimal biotinylation sequence of the biotin carboxy carrier protein (BCCP), the 15 amino acid variant thereof called AviTagTM, and the 76 amino acid BioEaseTM sequence, all of which are biotinylated by the biotin protein ligase BirA at a central lysine residue. Streptags such as Streptag II and One-STrEP-tag (IBA BioTAGnology, IBA GmbH, Germany) may also be used.

Furthermore, the antigen-binding polypeptides of the present invention can also be coupled with enzymes (such as alkaline phosphatase) or with recognition sequences for enzymes (such as the above BirA biotinylation sequences). Coupling of different antigen-binding polypeptides, such as VHHs directed against different epitopes of CD73, is also possible to enhance the efficiency of binding to the target antigen. For example, two or more of the single domain VHH antibodies described above may be coupled to biotin via a BirA recognition sequence and subsequently bound to avidin or streptavidin, or to modified streptavidin such as neutravidin or streptactin, via the high affinity binding of biotin. Avidin and streptavidin are tetrameric molecules that can bind four molecules of D-biotin. Such multimerised, tetravalent VHH single domain antibodies have an increased affinity for CD73. Streptavidin-coated beads, e.g. magnetic or Sepharose beads, can be used for multimerisation. Suitable beads are e.g. Strep-Tactin Magnetic Nanobeads (IBA BioTAGnology, IBA GmbH, Germany). The polypeptides of the present invention, and in particular the VHH single domain antibodies can of course also be coupled to immunoglobulins, e.g. to the Fc region of an immunoglobulin. In addition, the VHH single domain antibodies of the present invention can be prepared in the form of fusion proteins that comprise more than one VHH domain that binds to CD73, such as 2, 3, 4 or 5 VHH domains, which are expressed as a single protein chain. The additional VHH domains of the fusion protein may bind to the same or to different epitopes of CD73. Alternatively, the additional VHH domains of the fusion protein may bind to other target molecules, e.g. to CD73, CD4, CD8, CD19, CD20, CD56 and/or CD203a, or to immune checkpoint proteins such as PD-1, PD-L1, CTLA-4, LAG3, TIM3, TIGIT, PVR-IG, PVR or PVR-L2. In a particular preferred embodiment, the invention relates to a fusion protein that comprises a first VHH directed against CD73 and a second VHH directed against CD39.

In a particularly preferred aspect of the invention, the CD73-specific polypeptides described above, preferably those comprising one or more of the CDR regions listed SEQ ID NO:1-60 and SEQ ID NO:78-80 or modifications thereof as described above, and those comprising the sequences listed in SEQ ID NO:61-77 and SEQ ID NO:81-83 or immunologically active fragments or variants thereof as described above, are used for therapeutic and diagnostic purposes.

The antigen-binding polypeptides of the present invention are particularly suitable for use in a method of diagnosing a disease characterised by increased expression of CD73. The diagnosis is based on the detection of cells or tissues that express increased levels of CD73. These cells and tissues can be detected via a suitable label after binding one of the polypeptides according to the invention.

Thus, the present invention also relates to a method of diagnosing a disease characterised by increased cellular expression of CD73, said method comprising
(a) contacting a CD73-specific antigen-binding polypeptide as described above and defined in the claims with a biological sample from a patient under conditions that allow the formation of complexes of the antigen-binding polypeptide and CD73; and
(b) detecting the complexes of the polypeptide and CD73/CD73-expressing cells;
wherein detection of the complexes in step (b) indicates that the patient has a disease characterised by increased CD73 expression.

The disease to be diagnosed is preferably a hyperproliferative disease. It is particularly preferred that the disease is a solid tumor, such as gastric cancer, colon cancer, renal cancer, lung cancer, such as non-small cell lung carcinoma (NSCLC), head and neck cancer, breast cancer, or melanoma. It is also preferred that the disease is a haematological cancer disease, such as Burkitt's lymphoma, T-cell lymphoma, hairy cell leukaemia, chronic lymphocytic leukaemia (CLL), multiple myeloma, chronic myeloid leukaemia (CML), acute myeloid leukaemia (AML), and acute lymphoblastic leukaemia (ALL). In a particularly preferred embodiment of the invention, the method is used to diagnose a malignant non-Hodgkin's lymphoma, preferably multiple myeloma, CLL, CML, AML or ALL.

The biological sample may be a tissue sample from a patient, e.g. a tissue sample from a tumor. It may also be a sample of a body fluid, such as blood or lymph, or a bone marrow sample. For example, the sample may be a blood sample containing B lymphocytes and/or T lymphocytes. Depending on the disease to be diagnosed, it may also be a specific fraction of a body fluid. For example, in a procedure used to diagnose CLL, CML, AML or ALL, the fraction of the patient's blood or bone marrow containing B lymphocytes will be analysed.

The number of complexes detected in step (b) may be compared in the course of diagnosis with a reference sample analysed under identical conditions. The reference sample may be from a patient who has already been diagnosed with the disease to be detected, e.g. multiple myeloma, CLL, CML, AML or ALL. In this case, a measurement indicating an approximately identical number of complexes in the reference sample and the patient sample indicates that the patient is also likely to be afflicted with the disease. Alternatively, the reference sample may be a sample from a healthy individual. In this case, the disease can be detected in the patient if the measured number of complexes in the patient sample is significantly higher compared to the reference sample.

In another embodiment, the present invention relates to a method for visualizing CD73-expressing cells or tissues in a subject, comprising the step of
(a) administering to the subject a CD73-specific antigen-binding polypeptide as described above and defined in the claims, wherein the polypeptide is provided with a detectable label; and
(b) visualizing the CD73-expressing cells or tissues in the body by detecting the label.

The above method enables a so-called in vivo imaging of CD73-expressing cells or tissue in a subject, such as a human. The polypeptides administered to the subject are provided with a label that allows detection of the label without explantation of the CD73-expressing cells or tissues. Preferably, the label is a fluorescent label, such as an Alexa dye. Suitable Alexa dyes include, but are not limited to, Alexa-647 and Alexa-680. The amount of labelled polypeptide required for visualisation depends on various factors, such as the label as well as the weight of the subject to whom the polypeptide is administered. Quantities can generally be used as described below in the context of therapeutic treatment.

The CD73-specific antigen-binding polypeptide described hereinabove can also be used therapeutically to treat diseases or disorders which can be ameliorated by blocking the enzymatic activity of CD73. Adenosine triphosphate (ATP) plays a significant role in the regulation of immune responses. For example, ATP mediates pro-inflammatory responses by recruiting and activating inflammatory cells. CD73 catalyzes the hydrolyzation of ATP into adenosine monophosphate (AMP), and CD73 subsequently converts AMP into the nucleoside adenosine (ADO), the latter of which has immunosuppressive effects. It has been reported that CD73 and CD39 act as an immunological switch shifting an ATP-mediated pro-inflammatory environment to an ADO-mediated immunosuppressive environment.

In some diseases or conditions, CD73 and CD39 are balefully involved in the pathomecha-nism by suppressing an immune response. For example, it has been reported in the context with chronic infections, such as bacterial or viral infections, that CD73 and CD39 mediate immunosuppression resulting in persistence of the infection. Similarly, it was found that some tumors convert ATP into ADO through up-regulation of CD39 and CD73 expression on the tumor cells and regulatory immune cells, thereby preventing or suppressing an anti-tumor response. Accordingly, blocking of CD39 and/or CD73 is a reasonable approach for activating the immune response and overcoming immunosuppression in cancer diseases and chronic infections. In addition, blocking of CD39 and/or CD73 is useful for enhancing an immune response in a subject after vaccination.

Thus, another aspect of the present invention pertains to the antigen-binding polypeptides of the present invention for use in a method of treating a disease or disorder which is characterized by a pathologically enhanced level of CD73 activity. In a preferred embodiment, the disease to be treated is a solid tumor, such as gastric cancer, colon cancer, bladder cancer, glioma, glioblastoma, renal cancer, ovarian cancer, melanoma, prostate cancer, thyroid cancer, esophageal cancer, lung cancer, such as non-small cell lung carcinoma (NSCLC), head and neck cancer, breast cancer, and melanoma. In another preferred embodiment, the disease to be treated is a haematological cancer, such as a non-Hodgkin's lymphoma, multiple myeloma, chronic lymphocytic leukemia (CLL), chronic myeloid leukemia (CML), acute myeloid leukemia (AML) and acute lymphoblastic leukemia (ALL). In a more preferred embodiment of the invention, the disease to be treated is AML. In another preferred embodiment of the present invention, the tumor to be treated overexpresses CD73. In an even more preferred embodiment, the tumor to be treated overexpresses both CD39 and CD73.

In a preferred embodiment, the disease to be treated is a chronic infection. The infection can be caused by a viral, bacterial or eukaryotic pathogen. Chronic viral infections to be treated by the antigen-binding polypeptides of the present invention include infections with a human immunodeficiency virus (HIV), infections with a hepatitis C virus (HCV) and infections with a herpes virus. Chronic bacterial infections to be treated by the antigen-binding polypeptides of the present invention include infections with *Haemophilus influenzae*, *Pseudomonas aeruginosa*, *Moraxella catarrhalis*, *Streptococcus pneumoniae* and *Staphylococcus aureus.* Chronic eukaryotic infections to be treated by the antigen-binding polypeptides of the present invention include amoebic infections, such as an infection with *Entamoeba histolytica.*

The therapeutic effect of the polypeptides may be provided by binding of the antigen-binding polypeptide to CD73 and a subsequent reduction of the enzymatic activity of CD73 on the expressing cells. This means that the binding of the antigen-binding polypeptides of the invention preferably reduces the enzymatic activity of CD73 to about 80%, 70%, 60%, 50%, 40%, 30%, 20%, 10% or less of the activity of the enzyme measured in the absence of the respective antigen-binding polypeptide.

The therapeutic effect of the polypeptides may also be provided, at least in part, by killing CD73-expressing cells, in particular CD73-expressing tumor cells. For example, the killing of cells may result from antibody-dependent cell-mediated cytotoxicity (ADCC), complement-mediated cytotoxicity (CDC) and/or apoptotic processes which are induced by binding of the polypeptides of the invention to CD73. Alternatively, the polypeptides of the invention may be conjugated to one or more cytotoxic agents that result in cell death.

Preferably, binding of the antigen-binding polypeptide of the invention to CD73 leads to the death of at least 10%, 20%, 30%, 40%, 50%, 60%, 70%, 80% or even 90% or more of the CD73-expressing tumor cells of a population.

The CD73-specific antigen-binding polypeptides described above may be used in combination with other active ingredients, such as checkpoints inhibitors. Immune checkpoints, such as the cytotoxic T-lymphocyte-associated antigen 4 (CTLA-4) and programmed death 1 (PD-1), are negative regulators of the T-cell immune function. Inhibition of these checkpoints results in activation of the immune system and therefore has become a valuable immunotherapeutic approach for treating different types of cancer. It has been reported that during the treatment of cancer with checkpoint inhibitors, CD73 expression on T cells is increased as an escape mechanism. Accordingly, the administration of the CD73-specific antigen-binding polypeptides of the invention to cancer patient which are treated with checkpoint inhibitors is highly effective. Preferably, the CD73-specific antigen-binding polypeptides described above is used in combination with a checkpoint inhibitor selected from the group consisting of an inhibitor of CTLA-4, an inhibitor of PD-1, an inhibitor of CD274 (programmed cell death 1 ligand 1, PD-L1), an inhibitor of T cell immunoglobulin and ITIM domain (TIGIT), an inhibitor of poliovirus receptor-related immunoglobulin domain-containing (PVRIG), an inhibitor of poliovirus receptor (PVR), an inhibitor of poliovirus receptor-related 2 (PVRL2), and an inhibitor of T-cell immunoglobulin and mucin domain 3 (Tim-3).

The CD73-specific antigen-binding polypeptides disclosed herein may be used in combination with chemotherapy. Specifically, the polypeptides may be used in combination with one or more commonly known chemotherapeutic agents. Chemotherapeutic or cytotoxic agents which is suitable for being co-administered with the polypeptides of the present inventions include, but are not limited to, actinomycin, amsacrine, aminopterin, anthracycline, 5-azacytidine, 6-azauridine, azathioprine, bendamustine, biricodar, bleomycin, bortezomib, bryostatin, busulfan, calyculin, capecitabine, carmofur, carboplatin, chlorambucil, cisplatin, cladribine, clofarabine, cytarabine, dacarbazine, dasatinib, daunorubicin, decitabine, discodermolide, docetaxel, doxorubicin, doxifluridine, epirubicin, eribulin, estramustine, etoposide, exatecan, floxuridine, fludarabine, fluorouracil, 5-FU, fotemustine, gemcitabine, hydroxyurea, idarubicin, ifosfamide, imiquimod, irinotecan, irofulven, ixabepilone, lapatinib, lenalidomide, lomustine, lurtotecan, mafosfamide, masoprocol, mechlorethamine, melphalan, mercaptopurine, mitomycin, mitoxantrone, nelarabine, nilotinib, oxaliplatin, oxazaphosphorine, paclitaxel, pentostatin, pixantrone, plicamycin, procarbazine, raltitrexed, rebeccamycin, rubitecan, salinosporamide A, satraplatin, swainsonine, tariquidar, taxane, tegafur-uracil, temozolomide, testolactone, thiotepa, thioguanine, topotecan, trabectedin, tretinoin, triplatin, tetranitrate, and derivatives, tautomers and pharmaceutically active salts of the above compounds.

The CD73-specific antigen-binding polypeptides disclosed herein may be used in combination with radiotherapy. As used herein, radiation therapy or radiotherapy refers to the use of ionizing radiation for the targeted destruction of cancer cells or tissues. Radiation therapy includes various techniques for irradiating cancer tissue in a subject with high-energy radiation to destroy the cancer tissue. In the context of the invention, radiation therapy can include photon-based radiation, such as x-rays and gamma rays, and/or particle radiation, such as electron, carbon or proton beams. Preferably, radiation is performed as an external beam radiation therapy. The dose of radiation to be used in combination with the CD73-specific antigen-binding polypeptides of the invention will depend on the type of tumor to be treated and will normally be in a range of 20-75 Gy, preferably 30-60 Gy and more preferably 45-60 Gy. In a preferred aspect, the daily dose of radiation that is administered is 1.5-1.8 Gy for a child or adolescent and 2.0-2.5 Gy for an adult. Radiation therapy can be applied either before, simultaneously with, or after administration of the CD73-specific antigen-binding polypeptide.

In one embodiment, the CD73-specific antigen-binding polypeptides described above are used in combination with other antigen-binding polypeptides which are specific for other structures expressed on the surface of CD73-expressing cells. The binding of CD73-specific and other specific polypeptides to the cell may lead to a particularly efficient therapeutic effect, e.g. reduction of tumor cells. For example, the CD73-specific antigen-binding polypeptides may be used in combination with CD39-specific and/or CD203a-specific antigen-binding polypeptides, such as VHHs which are directed against CD39. Further, the CD73-specific antigen-binding polypeptides may be used in combination with other compounds that block or target other tumor markers, such as CD38, CD138, BCMA, CD229, CD203a to induce ADCC and/or CDC.

Preferably, the antigen-binding polypeptide of the present invention will have a size that results in physicochemical properties suitable for such therapeutic application, e.g. good solubility and tissue penetration, thermal stability and similar properties. Preferably, the antigen-binding polypeptide of the present invention used for therapeutic treatment of a patient will have a size of 80-250 amino acids, wherein a size of 90-200 amino acids, 90-180 amino acids, 90-150 amino acids, 100-140 amino acids and 100-130 amino acids is preferred.

In a further aspect, the invention provides a method for treating a disease or disorder which is characterized by a pathologically enhanced level of CD73 activity. The treatment comprises administering one or more of the polypeptides of the invention to a patient which suffers from one of the diseases mentioned above, in particular cancer or a chronic disease. Such therapeutic treatment includes both treatment of an acute disease state and prophylactic treatment to prevent disease.

The CD73-binding polypeptide will preferably be administered in the form of a pharmaceutical composition. Accordingly, the invention also provides a pharmaceutical composition comprising an antigen-binding polypeptide of the present invention. The antigen-binding polypeptide is thereby administered to the patient in a therapeutically effective amount, i.e. in an amount sufficient to significantly reduce the effect mediated by CD73 in the context of the disease and/or the number of CD73-expressing cells. In a preferred embodiment of the invention, the antigen-binding polypeptide is administered in an amount that results in a significant reduction of the CD73 activity in the patient. In another preferred embodiment of the invention, the antigen-binding polypeptide is administered in an amount that results in a substantially complete reduction of CD73-expressing cells. The reduction of CD73 activity or CD73-expressing cells may be local, e.g. in a tumour. The reduction of cells will preferably give rise to an improvement of one or more symptoms of the disease.

In yet another aspect, the present invention relates to the antigen-binding polypeptides of the present invention for use in a method of enhancing an immune response in a subject in need thereof. In yet another aspect, the present invention relates to the antigen-binding polypeptides of the present invention for use in a method of reversing immunosuppression in a subject in need thereof.

The exact amount of polypeptide that needs to be administered to achieve a therapeutic effect depends on several parameters. Factors relevant to the determination of the amount of polypeptide to be administered include, for example, the route of administration of the polypeptide, the size of the polypeptide in question, the nature of the cytotoxic residue (if any), the nature and severity of the disease, the medical history of the patient to be treated, and the age, weight, height and health status of the patient to be treated. A therapeutically effective amount of the antigen-binding polypeptide can be readily determined by one skilled in the art based on the common knowledge and the present disclosure.

The polypeptide is preferably administered to a subject in an amount sufficient to achieve a plasma concentration of from about 0.05 µg/ml to about 150 µg/ml, preferably from about 0.1 to about 50 µg/ml, more preferably from about 1 µg/ml to about 20 µg/ml, and typically from about 1 µg/ml to about 10 µg/ml, e.g. 5 µg/ml. The weekly dose may range from about 1 mg to about 500 mg of polypeptide per m² of body surface area of the patient. The weekly dose of the polypeptide may range from about 10-300 mg/m², preferably from about 100-200 mg/m², such as 150 mg/m². Depending on the size of the polypeptide, the amount of polypeptide to be administered can be adjusted.

The antigen-binding polypeptide of the present invention may be formulated for various modes of administration, e.g. oral administration as a tablet, capsule, powder, liquid or the like. However, it is preferred that the antigen-binding polypeptide is administered parenterally by intravenous injection or intravenous infusion. For example, administration may be by intravenous infusion, e.g. within 60 minutes, within 30 minutes, or within 15 minutes. It is further preferred that the antigen-binding polypeptide is administered locally by injection during surgery. Compositions suitable for administration by injection and/or infusion typically comprise solutions and dispersions, and powders from which such solutions and dispersions can be prepared. Such compositions will comprise the immunologically active polypeptide and at least one suitable pharmaceutically acceptable carrier. Suitable pharmaceutically acceptable carriers for intravenous administration include bacteriostatic water, Ringer's solution, physiological saline, phosphate buffered saline (PBS) and Cremophor EL^{™}. Sterile compositions for injection and/or infusion can be prepared by introducing the polypeptide in the required amount in a suitable carrier and then filtering it sterilely using a filter. Compositions for administration by injection or infusion should remain stable under storage conditions for a prolonged period after their preparation. The compositions may contain a preservative for this purpose. Suitable preservatives include chlorobutanol, phenol, ascorbic acid and thiomersal.

In one embodiment, the antigen-binding polypeptide of the present invention is formulated with carriers that protect the polypeptide from being excreted too rapidly from the body (sustained release formulations). Such formulations may include biocompatible polymers, such as ethylene vinyl acetate, polyanhydrides, polyglycolic acids, collagen, polyorthoesters and polylactic acids. Processes for the preparation of sustained-release formulations are sufficiently known in the prior art. These formulations may be provided in the form of semi-permeable films of hydrophobic polymers, e.g. in the form of microcapsules. The production of corresponding dosage forms as well as suitable excipients is described, for example, in Remington (2005).

In order to increase the therapeutic efficacy of the polypeptides according to the invention, it may be advantageous not only to block the action of CD73 on the surface of CD73-expressing cells, but also to kill these cells. This can be achieved, for example, by conjugating one or more cytotoxic agents to the antigen-binding polypeptides described herein.

The invention thus also provides a conjugate comprising as a first component an antigen-binding polypeptide as described above, preferably a polypeptide comprising one or more of the CDR regions listed in SEQ ID NO:1-60 and SEQ ID NO:78-80 or modifications thereof as described above, or a polypeptide comprising one of the sequences listed in SEQ ID NO:61-77 and SEQ ID NO:81-83 or immunologically active fragments or variants thereof as described above, and as a further component one or more cytotoxic residues. The coupling of the CD73-specific polypeptides to the cytotoxic agent can be carried out according to procedures customary in the state of the art.

Finally, the present invention also provides a kit for the detection of CD73 and CD73-expressing cells, the kit comprising an antigen-binding polypeptide as described above. The kit may further comprise other reagents and means necessary for the detection, e.g. reagents for visualising a label.

### BRIEF DESCRIPTION OF THE FIGURES

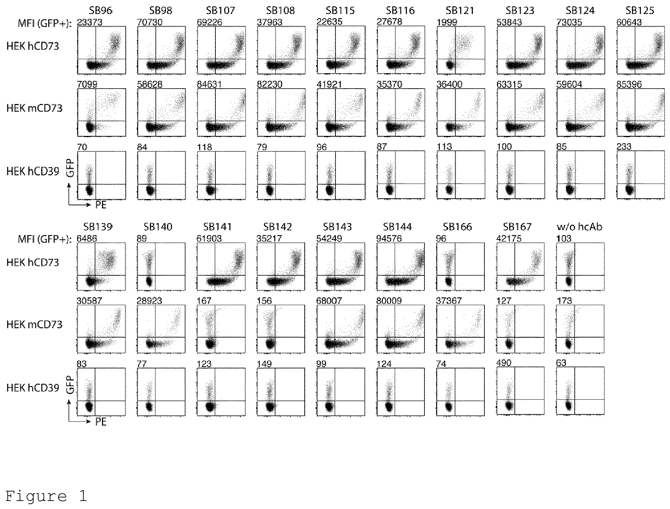

**Figure 1** shows the results of the analysis of hcAbs binding to CD73-transfected HEK cells by flow cytometry. HEK cells were co-transfected with expression vectors for green fluorescent protein (GFP) and human CD73, mouse CD73, and a control cell surface protein (CD39). 48h after transfection, cells were incubated with HEK cell supernatants containing the indicated rabbit IgG hcAbs. Bound antibodies were detected with PE-conjugated anti rabbit IgG secondary antibody.

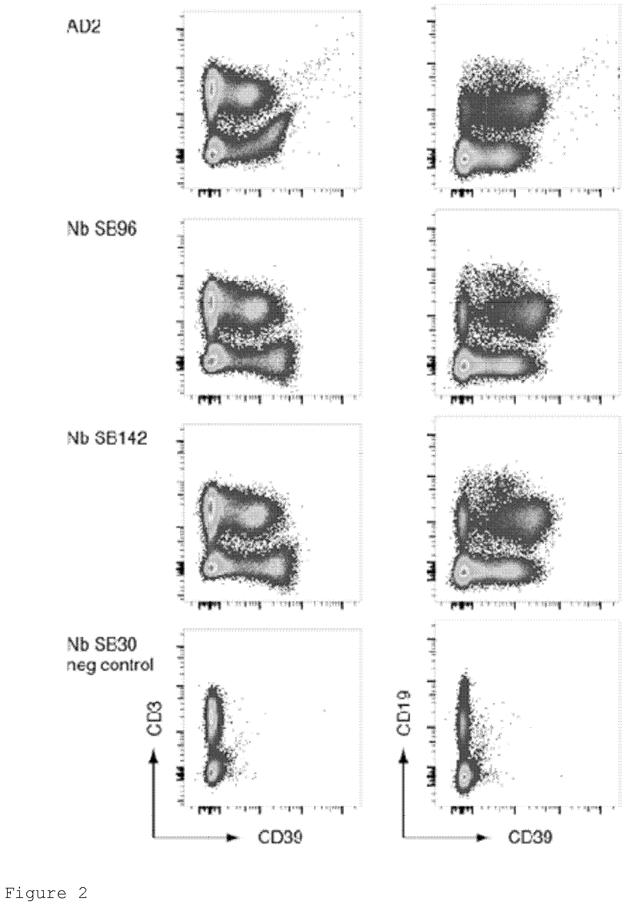

**Figure 2** shows the results of the analysis of hcAbs binding to native CD73 on blood leukocytes by flow cytometry

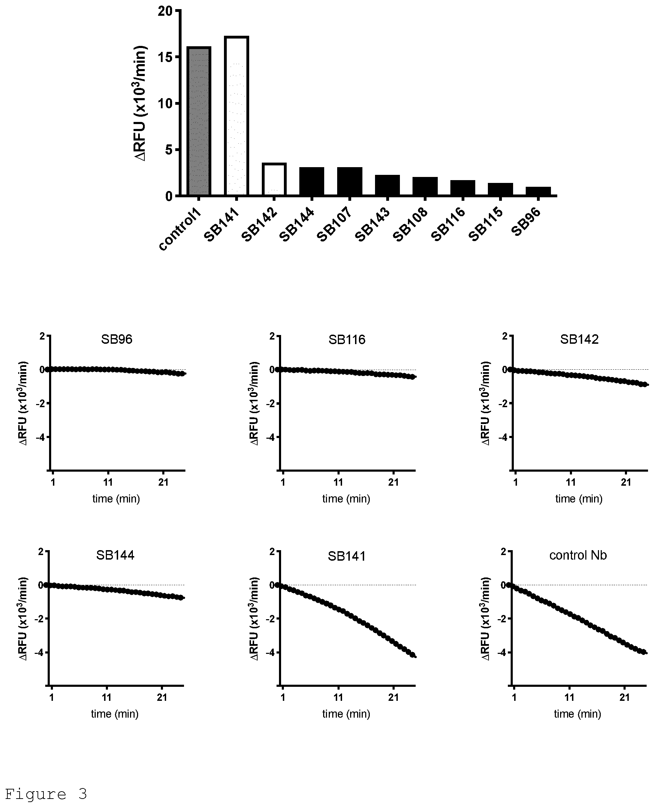

**Figure 3** shows the results of the measuring the effect of hcAbs on AMP-hydrolase activity of CD73. Recombinant human CD73 (50 ng) was pre-incubated for 30 min with hcAbs at RT in 96 well plates before addition of AMP and DPEA-Napht-Azide. Fluorescence signals were detected every 50 seconds for 30 repeats. ΔRFU values (× 10³/min) were calculated for a 10 min interval during the linear phase (from 5 min to 15 min) using Prism software (Graphpad). A non-specific rabbit IgG hcAb was used as control.

### EXAMPLES

The present invention is further described in more detail by the following examples which are only provided for illustrating the invention and which are not to be construed as limiting the scope of the invention. The following material and methods were used in the Examples.

### 1. Immunizations

1.1 Eukaryotic expression vectors for human CD73 (gene ID 4907) and mouse CD73 (gene ID 23959) were cloned into pCMV-SPORT6 after EcoRI and NotI digestion. The ectodomain of CD73 (aa 27-549) was expressed as a recombinant protein with a C-terminal His6x tag in transiently transfected HEK-cells and purified by immobilized metal affinity chromatography.

1.2 Two alpacas (designated SAL009 and SAL012) were immunized by a DNA-prime protein boost strategy as described previously (see Koch-Nolte et al. 2007). The humoral immune response was monitored in serially diluted serum on CHO cells transfected with the same plasmids used for immunization by IFM in 96 well microtiter plates on EVOS inverted fluorescence microscope. Animals were bled 7 days after the 3^{rd} or 4^{th} boost.

### 2. Phage display library construction

2.1 Mononuclear cells were isolated from 120 ml of blood by Ficoll-Paque^{™} (GE Healthcare, Chalfont St Giles, UK) gradient centrifugation. RNA purified from these cells by innuPREP RNA Mini Kit (Analytic Jena, Jena, Germany) was subjected to cDNA synthesis with alpaca CH2-specific primers. The nanobody coding region was amplified by PCR with degenerate nanobody-specific primers.

2.2 PCR products were purified from agarose gels, digested sequentially with SfiI and NotI (NEB, Ipswich, MA) and cloned into the pHEN2 phagemid vector downstream of the PelB-leader peptide and upstream of the chimeric His6x-Myc epitope tag (see Koch-Nolte et al. 2007). Transformation into XL1-Blue *E. coli* (Stratagene, La Jolla, CA) yielded libraries with sizes of 1-3 × 10⁶ clones.

2.3 Phage particles were precipitated with polyethylene glycol from culture supernatants of transformants infected with a 10-fold excess of M13K07 helper phage (GE Healthcare, Chalfont St Giles, UK). Panning of specific phage was performed in solution with CD39-transfected HEK cells. Phage particles (1 × 10¹¹) were incubated with CD39 transfected cells for 60 min with agitation at room temperature in DMEM (Gibco, ThermoFisher) with 2% BSA. Following extensive washing, bound phages were eluted from transfected cells by trypsinization.

2.4 Eluted phages were titrated and subjected to one or two more rounds of panning, following the same procedure. Phage titers were determined at all steps by infection of TG1 *E*. *coli* cells (Lucigen, WI). Plasmid DNA was isolated from single colonies and subjected to sequence analyses using pHEN2-specific forward and reverse primers.

### 3. Production of nanobodies and hcAbs

3.1 The coding region of selected nanobodies (Nbs) was subcloned using NcoI and NotI into the pCSE2.5 vector (Jäger et al. 2013) upstream of either a myc-C-hexahistiden tag or the hinge and Fc-domains of rabbit IgG, mouse IgG2c or human IgG1 (Eden et al. 2018). Recombinant myc-his tagged Nbs and rabbit IgG, mouse IgG2c, or human IgG1 heavy chain antibodies (hcAbs) were expressed in transiently transfected HEK-6E cells cultivated in serum-free medium. Six days post transfection, supernatants were harvested and cleared by centrifugation.

3.2 Nanobodies and hcAbs in cell supernatants were quantified by SDS-PAGE and Coomassie staining relative to marker proteins of known quantities: 10 µl samples of the supernatant were size fractionated side by side with standard proteins (albumin 4 µg, IgH 2 µg, IgL 1 µg, lysozyme 0.4 µg; albumin 1 µg, IgH 0.5 µg, IgL 0.25µg, lysozyme 0.1 µg). Yields of recombinant Nbs and hcAbs typically ranged from 0.5-3 µg/10µl.

3.3 Myc-His tagged Nbs were purified by immobilized metal affinity chromatography using Ni-NTA agarose (Sigma, St Louis, MO). Heavy chain antibodies proteins were purified by affinity chromatography using protein A-sepharose (GE-Healthcare).

### 4. Enzyme assay

4.1 CD73 catalyzes the hydrolysis of AMP to adenosine. This reaction can be monitored by the shift in fluorescence intensity of DPEA-Napht-Azide. This property was used to determine whether hcAbs could interfere with the enzymatic activity of CD73. Soluble recombinant human CD73 (50 ng) was pre-incubated for 30 min with Nbs or hcAbs (10 µl HEK-6E cell supernatant containing 10-100 µg/ml hcAb) at RT in 96 well plates before addition of AMP (Sigma) to a final concentration of 1 mM and of DPEA-Napht-Azide to a final concentration of 50 µM.

4.2 Fluorescence signals were recorded in white Nunc MicroWell plates (Nunc, 136101) on a Victor III plate reader (Perkin Elmer) (Exicitation filter: F485, Emission Filter: F535; CW-Lamp energy: 40000, counting time: 0.1 s). Data analyses were performed using Prism (Graphpad).

### 5. Flow cytometry

5.1 Untransfected HEK293 cells and HEK293 cells stably transfected with human or mouse CD73 were incubated for 30 min with HEK-6E cell supernatants containing rabbit IgG hcAbs (diluted 1:10 in PBS/0.1% BSA). Following washing with PBS/0.1% BSA, bound antibodies were detected with PE-conjugated goat anti-rabbit IgG polyclonal antibody (711-116-152 Dianova, Hamburg).

5.2 For dissociation analyses, two separate aliquots of CD73-transfected cells were incubated either with Cell Proliferation Dye eFluor 450 (eBioscience) or with hcAbs for 20 min at RT. Cells were washed four times, mixed at a 1:1 ratio and further incubated at RT for 120 min before staining of bound antibodies with PE-conjugated goat anti-rabbit IgG polyclonal antibody. The dissociation of hcAbs from the target cells and association with the eFluor 450 labeled cells was analyzed by flow cytometry using the FlowJo software (Treestar).

5.3 For epitope analyses, cells were preincubated with excess human IgG hcAbs (10 µl HEK-6E cell supernatant /100 µl PBS/0.1 % BSA) for 30 min at RT before addition of Nb-rabbit IgG hcAbs (1 µl HEK-6E cell supernatant /100 µl PBS/0.1 % BSA) and further incubation for 20 min at RT and staining with PE-conjugated anti-rabbit polyclonal antibody (711-116-152 Dianova, Hamburg). Cells were washed and analyzed by flow cytometry on a BD-FACS Canto. Data were analyzed using the FlowJo software (Treestar).

### Example 1: Induction of CD73-specific hcAbs

Two alpacas were immunized with CD73 for the induction of hcAbs. In particular, alpacas SAL009 and SAL012 were immunized and boosted with CD73 expression vectors by ballistic immunization essentially as described previously (Koch-Nolte et al 2007). Each alpaca received four DNA immunizations with administration intervals of two weeks. Each dose consisted of 12 shots of plasmid-conjugated gold particles (1 µg of DNA conjugated onto 0.5 mg gold particles per shot) applied with a pressure setting at 600 psi into the skin. Three weeks after the final genetic immunization, a single boost with 2 × 10⁷ CD73-transfected Hek293 cells was given. At regular intervals, blood samples were collected to monitor the induction of the humoral immune response over time. For the isolation of B-cells, blood was collected from these animals 7 days after the fourth DNA immunization and 7 days after the cell boost. Peripheral blood mononuclear cells were prepared from blood samples using Ficoll-Hypaque gradient centrifugation.

The induction of a humoral immune response and reactivity of the immune sera were confirmed by IFM. Compared to untransfected cells, CD73-transfected cells showed specific staining with the immune sera after detection of bound antibodies with PE-conjugated goat anti lama IgG secondary antibody (ab72485, Abcam, Cambridge, UK) (data not shown).

### Example 2: Selection VHHs from phage display libraries

To create a phage display library RNA was isolated from the peripheral blood lymphocytes of alpacas after the last injection using the innuPREP RNA Mini Kit (Analytic Jena, Jena) and converted into cDNA by reverse transcription. For reverse transcription a primer was used that binds to the CH2-domain of alpaca IgG2 and IgG3 isotypes. The coding region of the VHH was amplified by PCR and the VHH repertoire was cloned via specific restriction sites into the pHEN2 phagemid vector upstream of C-myc and Hexahistidine tags and the gp3 protein of the phage. The ligation products were then transformed into E. coli to create a phage library expressing VHH fragments. Phages were prepared according to standard protocols. DNA sequence analysis of 96 randomly selected clones confirmed the successful cloning of a broad VHH repertoire.

Selection of CD73-specific VHH from the immune phage libraries was performed using cell selections. Non-specific VHHs were removed via negative selection by incubation with 1 × 10⁷ non-transfected HEK293 cells. Two or three rounds of positive selection were then carried out on CD73-transfected HEK293 cells. After each selection round, selected clones were analyzed by DNA sequencing. Clones that were found more than once and clones with the same framework and a highly similar CDR3 carrying with or without additional amino acid substitutions were defined as a clone family. Comparative sequence analysis allowed grouping of the Nbs into 6 distinct families that bound human CD73, 5 of which bound to both, human and mouse CD73 (see Example 4). The sequences of the CDR3 are provided in Table 1. Members of a family share common CDR3 and framework sequences.

| **family** | **animal** | **varia nts** | **max diff** | **CDR3** | **specificity** | **name** | **clone** |
|---|---|---|---|---|---|---|---|
| **1** | SAL009 | 8 | 18 | **HK**YYYCS**N**K**VE**V**F**D**Y** | h+m | s+15 | SB96 |
| **2** | SAL012 | 2 | 1 | SRDLF**G**EGEYDY | h | s-12 | SB142 |
| **3** | SAL009 | 1 | 0 | IPGND | h+m | 1-5 | SB116 |
| **4** | SAL012 | 2 | 7 | TAGGP**D**YLGGRDI**V**YDY | h+m | 1-17 | SB144 |
| **5** | SAL009 | 1 | 0 | QRRWNAY | h+m | 1-7 | SB121 |
| **6** | SAL009 | 1 | 0 | YYY | h+m | 1-3 | SB139 |

- Table 1:: Families were characterized according to their binding to human (h) and mouse (m) CD73. Animal indicates immunized alpaca, variants indicates the number of clones carrying distinct, but evidently related amino acid sequences, max diff indicates the maximum difference between two members of a family in number of amino acid substitutions. Variant amino acid positions in the CDR3 within a family are indicated in bold. Names indicate the presence of a short (s) or long hinge (1), the absence (-) or presence (+) of a disulfide bond connecting CDR2 and CDR3, and the length of the CDR3 in numbers of amino acid residues.

### Example 3: Recombinant expression of VHH in HEK-6E cells

Representative VHHs of the above identified families were expressed as hcAbs comprising a rabbit IgG or as monomeric VHHs with C-myc and His6x tags in HEK-6E cells. The hcAbs were purified by Protein A affinity chromatography, the VHHs were purified by immobilized metal affinity chromatography. The elution buffer was exchanged to PBS by gel filtration and protein concentrations were determined by nanodrop. SDS-PAGE analysis confirmed the purity, integrity and concentration of the purified hcAbs and VHHs (not shown). The hcAbs and monomeric VHHs were purified with a yield between 0.1 and 100 mg per liter of culture.

### Example 4: Analysis of hcAbs binding to CD73-transfected HEK cells by flow cytometry

The binding specificities of the purified hcAbs were determined by flow cytometry using HEK cells transiently co-transfected with expression vectors for GFP and either mouse CD73, human CD73 or a control protein (CD39). 1×10⁶ cells were incubated with 10 µl of the Nb-rbIgG hcAb containing HEK-6E cell supernatants for 30 min at 4 °C. After washing, the cells were incubated with PE-conjugated anti-rabbit IgG polyclonal antibody (711-116-152 Dianova, Hamburg) and washed twice. The cells were resuspended in PBS and analyzed by flow cytometry on FACS Canto or FACS Celesta (BD Biosciences, Heidelberg). Data evaluation was conducted using the software FlowJo (Treestar, Stanford, US).

Results: The results are shown in Figure 1. It can be seen that hcAb of 4 VHH families showed binding to human CD73-transfected HEK293 cells, but not to untransfected cells, hcAbs of 6 VHH families showed binding to mouse CD73, three of these families showed specific reactivity with both, human CD73-transfecte cells and mouse-CD73 transfected cells.

### Example 5: Analysis of hcAbs binding to native CD39 on blood leukocytes by flow cytometry

Purified hcAbs were analysed for binding to native CD73 on the cell surface of peripheral blood leukocytes (PBMCs) by flow cytometry. To this end purified human PBMCs were incubated for 30 min at RT with 10 µl of rabbit hcAb-containing HEK-6E cell supernatants and bound antibodies were detected with a FITC conjugated anti rabbit IgG secondary antibody. To distinguish between B lymphocytes most of which express high levels of cell surface CD73, and T lymphocytes a subset of which express moderate levels of cell surface CD73 , cells were co-stained with PE-conjugated mouse anti-human CD3 mAb (UCHT1) and a PerCP/Cy5.5- conjugated mouse anti-human CD19 mAb (HIB19). After washing the cells, cells were analyzed by flow cytometry. A hcAb binding only to murine CD39 (SB30) was used as a negative control. As positive control, a FITC-conjugated monoclonal antibody against CD73 (clone AD2, Biolegend) was used. Gating was performed on lymphocytes on the basis of forward and side scattered light.

Results: The results are shown in Figure 2. The results show similar staining patters with hcAbs SB96 and SB142 and mAb AD2 on most B cells and on a subset of T cells.

### Example 6: Analysis of hcAbs binding to soluble CD39 by Biolayer interferometry

The binding off-rate constants of bivalent hcAbs to human and mouse CD73 protein were determined by bioluminescence kinetic analysis on Octet RED96 (ForteBio). Immobilization of rabbit IgG hcAbs on the protein A-biosensor surface was done in HEK-6E cell supernatant, and regeneration of analytes was done with 100 mM Glycin pH=2.5. Recombinant mouse or human CD73 ectodomain was used as analytes and tested at a saturating dose of 200 ng/µl. Sensorgrams were fitted using least square regression in association than dissociation Model in Prism8 (Graphpad). The results are depicted in Tables 2 and 3. Off-rates ranged from 2E⁻³ to 4 E⁻⁵ 1/s.

**Table 2: Off-rates for binding of soluble human CD73 to immobilized Nb-rbIgG hcAb assessed on Octet BLI.**

| VHH | Family | Kdis (s-1) |
|---|---|---|
| SB 96 | 1 | 3,9E-05 |
| SB 98 | 1 | 1,8E-03 |
| SB115 | 1 | 3,5E-04 |
| SB 107 | 1 | 1,8E-16 |
| SB 108 | 1 | 9,7E-05 |
| SB 123 | 1 | 4,3E-05 |
| SB 124 | 1 | 1,1E-03 |
| SB 125 | 1 | 5,8E-04 |
| SB 141 | 2 | 4,5E-04 |
| SB 142 | 2 | 3,6E-04 |
| SB 116 | 3 | 1,7E-04 |
| SB 143 | 4 | 1,3E-03 |
| SB 144 | 4 | 2,3E-04 |
| SB121 | 5 | 2,2E-02 |
| SB139 | 6 | 4,6E-02 |

### Example 7: Epitope analysis

To evaluate the epitopes on the CD73 protein recognized by different VHHs, representative rabbit IgG hcAbs were binned against a set of human IgG hcAbs, and bound hcAbs were detected with a PE-conjugated anti-rabbit IgG secondary antibody. Cells were pre-incubated with excess human IgG hcAbs for 20 min at RT to block specific epitopes before addition of rabbit IgG hcAbs as detectors and further incubation for 15 min at RT. Cells were washed, and bound antibodies were detected with PE-conjugated anti-rabbit IgG polyclonal antibody. Cells were washed and analyzed by flow cytometry on a BD-FACS Canto. Data were analyzed using the FlowJo software (Treestar). Lack of blockade by a non-binding control human IgG hcAb served as negative control (unblocked), and blockade of binding with the same VHH carrying human IgG hcAb as the rabbit IgG hcAb used for detection served as positive controls for blocking. Values for blocking were determined by comparing the mean fluorescence intensities (MFIs) of PE staining of transfected cells for unblocked vs blocked rabbit hcAbs using the formula: inhibition (%) = 100 - (MFI(blocked) / MFI(unblocked)^{∗}100). The values were rounded to whole percentages. MFI Values ranged between about 20 (strongest self-blockade) and about 15000 (strongest unblocked binding).

Results: The results of the epitope binning analyses with human CD73-expressing cells are summarized in Table 4. Human IgG hcAbs SB96, SB116 and SB143 (family 1, 3 and 4 respectively) blocked the binding of rabbit IgG hcAb SB96, SB116 and SB143 to human CD73-transfected HEK cells (designated epitope 1). In contrast, human IgG hcAb SB141 (family 2), SB139 (family 6), and SB 121 (family 5) primarily blocked the binding of the rabbit IgG hcAbs corresponding to their own clone (designated epitope 2, 3 and 4). The antibodies IPH5301 and MEDI9447 (developed by Innate Pharma and Medimmune respectively) reduce binding of Epitope 1-binding hcAbs much stronger than binding of hcAbs binding to Epitopes 2, 3 or 4, and can therefore be assigned to epitope 1.

**Table 3: Epitope assignment of human CD73-specific hcAbs. The human IgG hcAbs used for blocking are indicated on top. Rabbit hcAbs used for detection are indicated in the column "name". Numbers indicate percent inhibition of binding. Epitope indicates binding to distinct epitopes.**

| | | | Blocking hcAb | | | | | | | |
|---|---|---|---|---|---|---|---|---|---|---|
| | | | Fam.1 | Fam.3 | Fam. 4 | Fam. 2 | Fam. 6 | Fam.5 | | |
| Epitope | Family | name | SB96 | SB116 | SB14 3 | SB14 1 | SB13 9 | SB121 | IPH53 01 | MEDI 9447 |
| 1 | 1 | SB96 | 100 | 73 | 70 | 17 | -25 | -29 | 90 | 92 |
| 1 | 3 | SB116 | 100 | 72 | 65 | 47 | 2 | 6 | 94 | 97 |
| 1 | 4 | SB143 | 100 | 97 | 91 | 35 | 16 | 13 | 99 | 98 |
| 2 | 2 | SB141 | 13 | 16 | 66 | 84 | -7 | -1 | 40 | 26 |
| 3 | 6 | SB139 | 76 | 75 | 77 | 47 | 50 | 3 | 66 | 73 |
| 4 | 5 | SB121 | 53 | 75 | 85 | 77 | 0 | 69 | 77 | 74 |

### Example 8: CD73-specific VHH modulate the enzymatic activity of CD73

Soluble recombinant human CD73 (50 ng) was pre-incubated for 30 min with CD73-specific hcAbs or an irrelevant control hcAb (10 µl HEK-6E cell supernatant) at RT in 96 well plates before addition of AMP (Sigma) to a final concentration of 1 mM and of DPEA-Napht-Azide to a final concentration of 50 µM. Fluorescence signals were detected every 50 seconds for 30 repeats. Data analyses were performed using Prism (Graphpad).

Results: The results are shown in Figure 3. HcAb SB96 (family 1) and hcAb SB116 (family 3) effectively blocked hydrolysis of AMP while hcAb SB 141 (family 2) did not. Other hcAbs of family 1 (SB115, SB116, SB108, SB017) as well as hcAbs of family 4 (SB143, SB144) also inhibited CD73 catalyzed hydrolysis of AMP.

### LITERATURE

Eden et al. 2018. Front Immunol. 8:1989. PMID: 29410663
Goueli and Hsiao 2019. PLoS ONE 14: e0220094-19, PMID 31652269
Jäger et al. 2013 BMC Biotechnol. 13:52. PMID: 23802841
Koch-Nolte et al. 2007 FASEB J. 21:3490-8. PMID: 17575259
Remington: The Science and Practice of Pharmacy, Lippincott Williams & Wilkins; 21. ed (2005)

## Claims

1. Antigen-binding polypeptide comprising the CDR1, CDR2 and CDR3 region of a VHH domain of a camelid heavy chain antibody, **characterised in that** the polypeptide specifically binds to CD73, preferably human CD73.

2. Antigen-binding polypeptide according to claim 1, wherein said polypeptide inhibits tumor growth.

3. Antigen-binding polypeptide according to any of claims 1-2, **characterized in that** the polypeptide comprises the CDR sequences shown in SEQ ID NO:1-3, and preferably
(a) the CDR sequences shown in SEQ ID NO:4-6;
(b) the CDR sequences shown in SEQ ID NO:7-9;
(c) the CDR sequences shown in SEQ ID NO:10-12;
(d) the CDR sequences shown in SEQ ID NO:13-15;
(e) the CDR sequences shown in SEQ ID NO:16-18;
(f) the CDR sequences shown in SEQ ID NO:19-21,
(g) the CDR sequences shown in SEQ ID NO:22-24,
(h) the CDR sequences shown in SEQ ID NO:25-27,
or CDR sequences which differ from the CDR sequences shown in (a)-(h) in not more than one amino acid per CDR region.

4. Antigen-binding polypeptide according to any of claims 1-2, **characterized in that** the polypeptide comprises the CDR sequences shown in SEQ ID NO:28-30, and preferably
(a) the CDR sequences shown in SEQ ID NO:31-33;
(b) the CDR sequences shown in SEQ ID NO:34-36;
(c) the CDR sequences shown in SEQ ID NO:37-39;
or CDR sequences which differ from the CDR sequences shown in (a)-(c) in not more than one amino acid per CDR region.

5. Antigen-binding polypeptide according to any of claims 1-3, **characterized in that** the polypeptide comprises the CDR sequences shown in SEQ ID NO:40-42, and preferably
(a) the CDR sequences shown in SEQ ID NO:43-45;
(b) the CDR sequences shown in SEQ ID NO:46-48;
(c) the CDR sequences shown in SEQ ID NO:49-51;
(d) the CDR sequences shown in SEQ ID NO:52-54,
(e) the CDR sequences shown in SEQ ID NO:55-57,
(f) the CDR sequences shown in SEQ ID NO:58-60;
or CDR sequences which differ from the CDR sequences shown in (a)-(f) in not more than one amino acid per CDR region.

6. Antigen-binding polypeptide according to claim 5, **characterized in that** the polypeptide comprises an amino acid sequence selected from the group consisting of:
(a) the amino acid sequences of SEQ ID NO:61-77; and
(b) an amino acid sequence having at least 80% or more sequence identity to one of the amino acid sequences of SEQ ID NO:61-77.

7. Conjugate comprising an antigen-binding polypeptide according to any one of claims 1-6 coupled to a cytotoxic moiety.

8. Pharmaceutical composition comprising an antigen-binding polypeptide according to any one of claims 1-6 or a conjugate according to claim 7.

9. Antigen-binding polypeptide of any one of claims 1-6, conjugate of claim 7 or pharmaceutical composition of claim 8 for use in a method of treating a disease which is **characterized by** a pathologically enhanced level of CD73 activity, preferably a hyperproliferative disease, more preferably Burkitt's lymphoma, T-cell lymphoma, hairy cell leukemia, chronic lymphocytic leukemia (CLL), multiple myeloma, chronic myeloid leukemia (CML), acute myeloid leukemia (AML), acute lymphoblastic leukaemia (ALL), or a CD73-expressing solid tumour.

10. Nucleic acid molecule encoding an antigen-binding polypeptide according to any one of claims 1-6.

11. Expression vector comprising a nucleic acid molecule according to claim 10.

12. Host cell comprising a nucleic acid molecule according to claim 10 or an expression vector according to claim 11.

13. A method for the purification and/or concentration of CD73 and/or CD73-expressing cells in a biological sample, which comprises
(a) contacting an antigen-binding polypeptide according to any one of claims 1-6 with the sample under conditions that allow the formation of complexes of the polypeptide and CD73; and
(b) separating the complexes are from the sample.

14. A method for the detection of CD73 and/or CD73-expressing cells in a biological sample, comprising
(a) contacting an antigen-binding polypeptide according to any one of claims 1-6 with the sample under conditions that allow the formation of complexes of the polypeptide and CD73; and
(b) detecting the complexes in the sample.

15. A method for the diagnosis of a disease **characterised by** increased CD73 expression comprising
(a) contacting an antigen-binding polypeptide according to any one of claims 1-6 with a biological sample from a patient under conditions that allow the formation of complexes of the polypeptide and CD73; and
(b) detecting complexes of the polypeptide and CD73/CD73-expressing cells;
wherein detection of the complexes in step (b) indicates that the patient has a disease **characterised by** increased CD73 expression.
